(19) **Europäisches Patentamt / European Patent Office / Office européen des brevets**

(11) **EP 1 562 649 B1**

(12) **EUROPEAN PATENT SPECIFICATION**

(45) Date of publication and mention
of the grant of the patent:
**23.05.2007 Bulletin 2007/21**

(21) Application number: **03786673.8**

(22) Date of filing: **14.11.2003**

(51) Int Cl.:
**A61L 15/28** (2006.01)

(86) International application number:
**PCT/US2003/036149**

(87) International publication number:
**WO 2004/045662 (03.06.2004 Gazette 2004/23)**

(54) **ABSORBENT ARTICLES INCLUDING CHITIN-BASED FECES MODIFICATION AGENT**

ABSORBIERENDE ARTIKEL AUF BASIS VON CHITIN ZUR MODIFIZIERUNG VON FAECES

ARTICLES ABSORBANTS COMPRENANT UN AGENT DE MODIFICATION DE MATIERES FECALES A BASE DE CHITINE

(84) Designated Contracting States:
**AT BE BG CH CY CZ DE DK EE ES FI FR GB GR HU IE IT LI LU MC NL PT RO SE SI SK TR**

(30) Priority: **14.11.2002 US 294543**

(43) Date of publication of application:
**17.08.2005 Bulletin 2005/33**

(73) Proprietor: **THE PROCTER & GAMBLE COMPANY**
**Cincinnati, Ohio 45202 (US)**

(72) Inventors:
• **CARLUCCI, Giovanni**
**I-66100 Chieti (IT)**

• **MASON, Oliver, Edwin, Clarke**
**Mason, OH 45040 (US)**
• **ROE, Donald, Carroll**
**West Chester, OH 45069 (US)**
• **SCHMIDT, Mattias**
**65510 Idstein (DE)**

(74) Representative: **Kremer, Véronique Marie Joséphine et al**
**Procter & Gamble Service GmbH**
**Sulzbacher Strasse 40-50**
**65824 Schwalbach am Taunus (DE)**

(56) References cited:
EP-A- 0 761 867          EP-A- 1 149 593
WO-A-91/12029          WO-A-99/32697

**Description**

**FIELD OF THE INVENTION**

The present invention relates to articles which absorb and/or contain bodily exudates, including disposable absorbent articles such as diapers, adult incontinence products and the like. More particularly, the invention relates to disposable absorbent articles including one or more agents which act to modify the physical properties of feces or other bodily wastes which may be deposited in the article.

**BACKGROUND OF THE INVENTION**

The major function of absorbent articles such as diapers and adult incontinence briefs is to prevent body exudates from soiling, wetting, or otherwise contaminating clothing or other articles, such as bedding, that may come in contact with the wearer. In recent years, disposable diapers have become very popular and have generally replaced durable cloth absorbent articles because of their convenience and reliability. However, despite the effectiveness of such disposable absorbent articles, body exudates often still leak or are stored in the diaper such that the exudates soil and/or irritate the skin of the wearer. Additionally, body exudates often adhere aggressively to skin; increasing the difficulty of cleaning and increasing the likelihood of chronic residual contamination. The fundamental causes of these and other key problems with absorbent articles of the art lie in the mobility under applied shear stress and adhesiveness of the body exudates, especially feces.

The undesirable effects of leakage and/or improper containment, such as difficult cleanup and/or residual skin contamination are, especially evident with regard to fecal matter deposited in the diaper. Feces contained in the diaper can harm the skin of the wearer over time and feces leaking from the diaper almost invariably presents unpleasant, messy clean-ups. Thus, several attempts have been made to add features to diapers such as barriers, pockets, spacers, transverse barriers, apertured topsheets and the like to limit the movement of the fecal material across the topsheet and/or to better confine the fecal matter in the diaper. However, such attempts have been generally unsuccessful because they fail to address the fundamental causes of these problems (i.e., the properties of feces) and, because of their cost and complexity. Further, many of the means for isolating or containing feces are directed to fecal material with certain physical properties (e.g., viscosity, free water content and particle size) and are not effective with exudates with physical properties outside a very small range.

Accordingly, it would be desirable to provide an absorbent article with improved feces management properties. Further, it would be advantageous to provide an economical disposable article with the ability to minimize the negative effects of feces or other viscous bodily waste on the wearer or the caregiver. It would also be advantageous to provide an article which is designed to chemically or physically interact with the feces and to change the properties of the feces in order to improve acceptance of feces into the article and/or immobilization of the feces within the article and/or reduce the contamination of the wearer's skin with feces. Also, it would be desirable to provide an article having sufficient effective capacity and retention capability to store the physically or chemically modified feces safely and cleanly away from the wearer's skin and/or clothing throughout the expected time of use.

**BRIEF DESCRIPTION OF THE DRAWINGS**

While the specification concludes with claims which particularly point out and distinctly claim the present invention, it is believed that the present invention will be better understood from the following description of preferred embodiments, taken in conjunction with the accompanying drawing wherein:

Figure 1 is a schematic front view of an apparatus which may be used to measure Acceptance Under Pressure and Storage Under Pressure characteristics of structures.
Figure 2 is a top view of a standard storage element.

**SUMMARY OF THE INVENTION**

In accordance with a first aspect of the present invention, a disposable absorbent article adapted to receive feces is provided. The disposable absorbent article comprises:

an effective concentration of a chitin-based feces modifying agent disposed in the article such that the chitin-based feces modifying agent is available to contact at least a portion of the feces deposited in the article.

In accordance with a second aspect of the present invention, a disposable absorbent article adapted to receive feces having a first waist region, a second waist region opposed to the first waist region, a crotch region disposed between the first waist region and the second waist region is provided. The disposable absorbent article comprises:

(a) a liquid pervious topsheet;
(b) a liquid impervious backsheet joined to at least a portion of the topsheet;
(c) an absorbent core disposed between at least a portion of the topsheet and the backsheet; and
(d) an effective concentration of a chitin-based feces modifying agent disposed in the article such that the chitin-based feces modifying agent is available to contact at least a portion of the feces deposited in the article, whereby changing the viscosity or Hardness of at least some of the feces which may be deposited in the article.

In accordance with a third aspect of the present invention, a disposable absorbent article adapted to receive feces having a first waist region, a second waist region opposed to the first waist region, a crotch region disposed between the first waist region and the second waist region is provided. The disposable absorbent article comprises:

(a) a liquid pervious topsheet;
(b) a liquid impervious backsheet joined to at least a portion of the topsheet;
(c) an absorbent core disposed between at least a portion of the topsheet and the backsheet;
(d) a waste management element having an Acceptance Under Pressure value of greater than about 0.50 grams per square inch per milliJoule of energy; and
(e) an effective concentration of a chitin-based feces modifying agent.

All percentages, ratios and proportions are by weight, and all temperatures are in degrees Celsius (°C), unless otherwise specked. All measurements are in SI units, unless otherwise specified.

## DETAILED DESCRIPTION OF THE INVENTION

As used herein, the term "absorbent article adapted to receive feces" ("absorbent articles") refers to devices which absorb and contain body exudates and, more specifically, refers to devices which are placed against or in proximity to the body of the wearer to absorb and contain the various exudates, including but not limited to feces, discharged from the body. The term "disposable" is used herein to describe absorbent articles which generally are not intended to be laundered or otherwise restored or reused as absorbent articles (i.e., they are intended to be discarded after a single use and, preferably, to be recycled, composted or otherwise discarded in an environmentally compatible manner). As used herein, the term "diaper" refers to an absorbent article generally worn by infants and incontinent persons about the lower torso. The present invention is also applicable to other absorbent articles such as incontinence briefs, incontinence undergarments, absorbent inserts, training pants, diaper holders and liners, and the like.

In one embodiment of the present invention the absorbent article is a disposable diaper. Typically, modern disposable diapers comprise a liquid pervious topsheet a liquid impervious backsheet; an absorbent core which is preferably positioned between at least a portion of the topsheet and the backsheet; side panels; elasticized leg cuffs; an elastic waist feature; and a fastening system. In one embodiment opposing sides of the disposable diaper may be seamed or welded to form a pant. This allows the article to be used as a pull-on type diaper, such as a training pant. Additional illustrative, but non limiting, information on construction, assembly, and the various components of disposable diapers may be found in U.S. Pat. No. 3,860,003 to Buell; U.S. Pat. No. 5,151,092 to Buell; U.S. Pat. No. 5,221,274 to Buell; U.S. Pat. No. 5,554,145 to Roe et al. on September 10, 1996; U.S. Pat. No. 5,569,234 to Buell et al.; U.S. Pat. No. 5,580,411 to Nease et al.; U.S. Patent No. 6,004,306 to Robles et al.; U.S. Patent No. 5,938,648 to LaVon et al.; U.S. Pat. No. 5,865,823 to Curro; U.S. Pat. No. 5,571,096 to Dobrin et al.; U.S. Patent No. 5,518,801 to Chappell, et al.; U.S. Patent 4,573,986 to Minetola et al.; U.S. Pat. No. 3,929,135, to Thompson; U.S. Pat. No. 4,463,045 to Ahr, et al.; U.S. Pat. No. 4,609,518 to Curro et al.; U.S. Pat. No. 4,629,643 to Curro et al.; U.S. Pat. No. 5,037,416 to Allen et al.; U.S. Pat. No. 5,269,775 to Freeland et al.; U.S. Patent 4,610,678 to Weisman et al.; U.S. Patent 4,673,402 to Weisman et al.; U.S. Patent 4,888,231 to Angstadt; U.S. Pat. No. 5,342,338 to Roe; U.S. Pat. No. 5,260,345 to DesMarais et al.; U.S. Pat. No. 5,026,364 to Robertson; U.S. Patent 3,848,594 to Buell; U.S. Patent 4,846,815 to Scripps; U.S. Patent 4,946,527 to Battrell; U.S. Pat. No. 4,963,140 to Robertson et al.; U.S. Pat. No. 4,699,622 to Toussant et al.; U.S. Pat. No. 5,591,152 to Buell et al.; U.S. Patent 4,938,753 to Van Gompel, et al.; U.S. Patent No. 5,669,897 to LaVon, et al.; U.S. Patent No. 4,808,178 to Aziz et al.; U.S. Patent No. 4,909,803 to Aziz et al.: U.S. Pat. No. 4,695,278 to Lawson and U.S. Patent No. 4,795,454 issued to Dragoo.

In one alternative embodiment of the present invention a portion of the absorbent article, such as part or all of the topsheet, part or all of the barrier leg cuffs and the like, may be optionally coated with a lotion, as is known in the art. Examples of suitable lotions include, but are not limited to, those described in U.S. Pat. Nos. 5,607,760 to Roe on;

U.S. Pat. No. 5,609,587 to Roe; U.S. Pat. No. 5,635,191 to Roe et al.; U.S. Pat. No. 5,643,588 to Roe et al.; and U.S. Pat. No. 5,968,025 to Roe et al..

Chitin-based feces modifying agent

The absorbent article of the present invention includes one or more chitin-based feces modifying agent ("FMAs", "viscous bodily waste modifying agents", or "modifying agents") in an effective concentration capable of modifying the chemical or physical properties of viscous bodily waste, such as feces. As used herein, "chitin-based feces modifying agent" (or FMA) refers to any chitosan material capable of increasing the hardness of a given fecal analog, or preferably actual feces, by at least about 100% as measured by the Hardness Method, described herein. However, depending on the particular article design and the type of feces, embodiments are contemplated which increase the effective viscosity of feces, increase the ease of dewatering the feces, decrease the stickiness of the feces, decrease the adhesion characteristics of the feces, or any combination of the above. Although the feces modifying agents of the present inventions may be capable of modifying the properties of solid feces, the FMAs are generally most effective in altering the properties of viscous fluid feces which generally have a viscosity of greater than about 10 cP and less than about $10^7$ cP at a shear rate of one 1/sec, (at about 35 °C), and more particularly between about $10^2$ cP and $10^7$ cP at a one 1/sec shear rate, in a controlled stress rheometry test using parallel plates on a controlled stress rheometer. (For reference, water has a viscosity of 1.0 cP at 20 °C and JIF CREAMY peanut butter (available from the Procter & Gamble Co., Cinti., OH) has a viscosity of approximately 4 X $10^5$ cP at 25 °C at this same shear rate). The method for determining viscosity, as used herein, is described in detail in the Test Methods section below.

As used herein, FMA refers to any chitosans, modified chitosans, crosslinked chitosans chitosan salts and other chitosan-based materials.

While not wanting to be limited by theory, it is believed that the chitin-based feces modifying agents may function as thickeners, generally increasing the structure of the feces by increasing the degree of water binding, leading to an increase in viscosity, reducing mobility of the feces. Furthermore, it is also believed that chitin-based feces modifying agents may also function as ionic complexing agents which may complex with chemical entities in the feces to form regions of increased structure and rigidity within the feces. The resultant complex acts to stabilize or bind water more tightly in the feces. Finally, it is also believed that chitin-based feces modifying agents may increase the viscosity of the feces by dissolving in the free water in the feces and osmotically "binding" water, thereby increasing the solid "structure" of the feces.

Regardless of the specific effect of the FMA on feces, the FMA must be available to the feces in order to perform its function. As used herein, in the context of FMAs, the term "available" indicates that the FMA is positioned within the absorbent article or presented by the absorbent article or a component of the absorbent article during the course of normal wearing of the absorbent article so as to directly contact at least a portion of the feces deposited in the absorbent article or on the wearer's skin. If the FMA is positioned within a structure (e.g., in an absorbent layer under a topsheet), the topsheet must be substantially penetrable by the feces. In such cases, the FMA is "available" if the structure has an Acceptance Under Pressure greater than about 0.50 grams per square inch per milliJoule of energy, and preferably greater than about 1.0 grams per square inch per milliJoule of energy, as measured by the Acceptance measurement described in the Methods section below. If the FMA is encapsulated, it should be released by the absorbent article at or about the time when the feces insults the absorbent article. For example, the FMA may be retained by a water-soluble film which, upon contact with urine or fecal water, dissolves and releases the FMA to contact the feces.

An "effective concentration" of an FMA, as used herein, refers to the relative amount of the FMA required to have a measurable effect on the Hardness (as measured by the Hardness Method described below) of at least a portion of the feces in the absorbent article or on the skin of the wearer. Data illustrating an "effective concentration" are provided below. Preferably, a concentration of an FMA of at least about 0.01 weight percent of the feces to be treated is desirable, and more typically between about 0.1 and about 50 weight percent of the feces is available to the feces. For example, to treat an entire 25 gram feces loading in a diaper (i.e., a "bulk" treatment) at a 5 weight percent level, 1.25 grams of the FMA must be available to the fecal mass (assuming the specific gravity of the feces is 1.0). Thus, the FMA is preferably present in the absorbent article in concentrations ranging from about 0.001% to about 50% by weight of the article, more preferably the concentration is ranging from about 0.01% to about 20% by weight of the article.

The FMA is preferably capable of increasing the Hardness by about 100% at a concentration of no more than about 20 weight percent of the feces to be treated at room temperature (20-25°C). More preferably, the FMA is capable of increasing the Hardness by about 100% at a concentration of no more than about 10 weight percent of the feces to be treated. Even more preferably, the FMA is capable of increasing the Hardness by about 100% at a concentration of no more than about 5 weight percent of the feces to be treated. In other preferred embodiments, the FMA is capable of increasing the Hardness by about 100% at a concentration of no more than about 1 weight percent of the feces to be treated. In yet other preferred embodiments, the FMA is capable of increasing the Hardness by about 100% at a concentration of no more than about 0.5 weight percent of the feces to be treated. Typically, the FMA is capable of increasing the Hardness by about 100% at a concentration of between about 0.1 and about 10 weight percent of the feces to be treated.

Preferably, the defined increase in Hardness is effected within the range of between about 30 minutes, more preferably within about 15 minutes, even more preferably within about 5 minutes, even more preferably within about 3 minutes, and most preferably in about 1 minute after contact with the feces. Typically, the desired Hardness change is effected within the range of about 1 minute to about 10 minutes. In more preferred embodiments, the defined increase in Hardness is effected within about 3 minutes at an FMA concentration of no more than about 5% by weight of the feces to be treated or within 3 minutes at an FMA concentration of about 1.5% by weight of the feces to be treated. In other preferred embodiments, the FMA is capable of increasing the Hardness of a fecal analog, or actual feces, by about 200% within about 3 minutes at a concentration of no more than about 5%. In yet other preferred embodiments, the FMA is capable of increasing the Hardness of a fecal analog, or actual feces, by about 400% within about 3 minutes at a concentration of no more than about 5%.

Chitosan is a partially or fully deacetylated form of chitin, a naturally occurring polysaccharide. Chitosan is an aminopolysaccharide typically prepared by deacetylation of chitin (poly-beta(1-4)-N-acetyl-D-glucosamine) and is structurally similar to cellulose, except that the C-2 hydroxyl group in cellulose is substituted with a primary amine group in chitosan. Chitin occurs widely in nature, for example, in the cell walls of fungi and the hard shell of insect and crustaceans.

Methods for the manufacture of chitosan are well known. Generally, chitin is milled into a powder and dematerialized with an organic acid, such as acetic acid. Proteins and lipids are then removed by treatment with a base, such as sodium hydroxide, followed by chitin deacetylation by treatment with concentrated base, such as 40 % sodium hydroxide.

The properties of chitosan relate to its polyelectrolyte and polymeric carbohydrate character. Thus, it is generally insoluble in water, in alkaline solutions at pH level of above about 6.5, or in organic solvents. It generally dissolves readily in dilute solutions of organic acids such as formic, acetic, tartaric, glycolic, lactic and citric acids, and also in dilute mineral acids, except, for example, sulfuric acid. In general, the amount of acid required to dissolve chitosan is approximately stoichiometric with the amino groups. Since the pKa for the amino groups present in chitosan is between 6.0 and 7.0, they can be protonated in very dilute acids or even close to neutral conditions, rendering a cationic nature to this biopolymer.

Suitable chitosan materials for use herein include both water-soluble and water insoluble chitosan. As used herein, a material will be considered to be water-soluble when it substantially dissolves in excess water to form a clear and stable solution, thereby, losing its initially particulate form and becoming essentially molecularly dispersed throughout the water solution. Preferred chitosan materials for use herein are water soluble, i.e., at least 0.5 gram, preferably at least 1 gram and more preferably at least 2 grams of the chitosan materials are soluble in 100 grams of water at 25°C and one atmosphere. By "solubility" of a given compound it is to be understood herein the amount of said compound solubilized in de-ionized water at 25°C and one atmosphere in absence of precipitate.

Chitosan materials (i.e., chitosan, chitosan salts, modified chitosans, cross-linked chitosan and other chitosan-based materials) may generally have a wide range of molecular weights. Chitosan materials with a wide range of molecular weights are suitable for use in the present invention. In one embodiment of the present invention the chitosan material for use herein has a molecular weight ranging from about 1,000 to about 10,000,000 grams per gram moles and more preferably from about 2,000 to about 1,000,000. Molecular weight means weight average molecular weight. Methods for determining the weight average molecular weight of chitosan materials are known to those skilled in the art. Typical methods include, for example, light scattering, intrinsic viscosity and gel permeation chromatography. It is generally most convenient to express the molecular weight of a chitosan material in terms of its viscosity in a 1.0 weight percent aqueous solution at 25°C with a Brookfield viscometer using the cylindrical # 64 spindle. It is common to indirectly measure the viscosity of the chitosan material by measuring the viscosity of a corresponding chitosan salt, such as by using a 1.0 weight percent acetic acid aqueous solution. Chitosan materials suitable for use in the present invention will suitably have a viscosity in a 1.0 weight percent aqueous solution at 25°C of from about 1 centipoise to about 80,000 centipoise, more suitably from about 30 centipoise to about 10,000 centipoise, even more suitably from 50 centipoise to about 1,000 centipoise and even more suitably from 100 centipoise to about 500 centipoise.

The pH of chitosan materials depends on the process used to prepare the chitosan material. Preferred chitosan materials for use herein have an acidic pH, typically in the range of about 4 to about 6, more preferably from 4 to 5.5 and even more preferably from about 4.5 to about 5. Highly preferred pH is around about pH 5, which corresponds to the skin pH. By pH of chitosan material it is meant herein the pH of a 1 % chitosan solution (1gram of chitosan material dissolved in 100 grams of distilled water) measured by a pH-meter at 25°C and 1 atmosphere.

In one embodiment of the present invention the chitosan materials for use herein are chitosan salts, especially water-soluble chitosan salts. A variety of acids can be used for forming chitosan salts. Suitable acids for use are soluble in water or partially soluble in water, are sufficiently acidic to form the ammonium salt of chitosan and yet not sufficiently acidic to cause hydrolysis of chitosan, and are present in amount sufficient to protonate the reactive sites of chitosan.

Examples of chitosan salts formed with an inorganic acid include, but are not limited to, chitosan hydrochloride, chitosan hydrobromide, chitosan phosphate, chitosan sulphonate, chitosan chlorosulphonate and mixtures thereof. Examples of chitosan salts formed with an organic acid include, but are not limited to, chitosan formate, chitosan acetate, chitosan lactate, chitosan glycolate, chitosan malonate, chitosan epoxysuccinate, chitosanbenzoate, chitosan chloroacetate, chitosan adipate, chitosan citrate, chitosan salicylate, chitosan propionate, chitosan nitrilotriacetate, chitosan itaconate, chitosan hydroxyacetate, chitosan butyrate, chitosan isobutyrate, chitosan acrylate, and mixtures thereof. It is also suitable to form a chitosan salt using a mixture of acids including, for example, both inorganic and organic acids.

Some chitosan salts suitable for use herein include those formed by the reaction of chitosan with an amino acid. Amino acids are molecules containing both an acidic and amino functional group. The use of amino acids instead of other acids is highly preferred as those chitosan amino salts have higher skin compatibility. Indeed most of the amino acids are naturally present on the skin. Chitosan salts of pyrrolidone carboxylic acid are effective moisturizing agents and are non-irritating to skin. Amino acids for use herein include both linear and/or cycloamino acids. Examples of amino acids for use herein include, but are not limited to, alanine, valine, leucine, isoleucine, proline, phenylalanine, triptophane, methionine, glycine, serine, cysteine, tyrosine, asparagine, glutamine, aspartic acid, glutamic acid, lysine, arginine, histydine, hydroxyproline and the like. One suitable example of cyclo amino acid is pyrrolidone carboxylic acid, which is a carboxylic acid of pyrrolidone.

Some preferred chitosan salts include, chitosan pyroglutamate salt, which is a mixture of chitosan and pyroglutamic acid; chitosan hydrochloride; and chitosonium pyrrolidone carboxylate, which is the chitosan salt of 2-pyrrolidone carboxylic acid. Reference is made to WO87/7618 filed on December 12, 1987 by Union Carbide Corporation, which describes in details processes for the preparation of such chitosan salts.

Other chitosan material are suitable for use herein include cross-linked chitosans and modified chitosans. Crosslinking agents suitable for use in the present invention are generally water-soluble and do not substantially reduce the fecal modifying properties of chitosan. One suitable crosslinking agent is an organic compound having at least two functional groups or functionalities capable of reacting with active groups located on the chitosan materials. Examples of such active groups include, but are not limited to, carboxylic acid (-COOH), amino (-NH$_2$), or hydroxyl (OH) groups. Examples of such suitable crosslinking agents include, but are not limited to, diamines, polyamines, diols, polyols, polycarboxylic acids, polyoxides and combinations thereof. One way to introduce a crosslinking agent with the chitosan solution is to mix the crosslinking agent with chitosan during preparation of the solution. Another suitable crosslinking agent comprises a metal ion with more than two positive charges, such as Al$^{3+}$, Fe$^{3+}$, Ce$^{3+}$, Ce$^{4+}$, Ti$^{4+}$, Zr$^{3+}$, Cr$^{3+}$ and combinations thereof.

In the embodiment herein where crosslinking agents are used, a suitable amount of crosslinking agent is from about 0.001 to about 30 weight percent based on the total dry weight of chitosan used to prepare the crosslinked-chitosan, more specifically from about 0.02 to about 20 weight percent, more specifically from about 0.05 to about 10 weight percent and most preferably from about 0.1 to about 5 weight percent.

Modified chitosans for suitable use herein include any chitosan where the glucan chains carry pendant groups. Examples of such modified chitosans include, but are not limited to, carboxymethyl chitosan, methyl pyrrolidone chitosan, glycol chitosan and the like. Methylpyrrolidone chitosan is described in US Pat No. 5,378,472 issued on January 3, 1995 to Muzzarelli, and water-soluble glycol chitosan and carboxymethyl chitosan are described in WO87/07618 filed on December 12, 1987 by Union Carbide Corporation. Particularly suitable modified chitosans for use herein include water-soluble covalently bonded chitosan derivatives or ionically bonded chitosan derivatives obtained by contacting salt of chitosan with electrophilic organic reagents. Such water-soluble chitosan derivatives are described in US Pat No. 5,621,088 to Gruber and US Pat No. 5,597,811 to Gruber.

Suitable electrophilic organic reagents suitable for use for the preparation of chitosan derivatives contain from 2 to 18 carbon atoms or more per molecule and typically from 2 to 10 carbon atoms per molecule. In addition the electrophilic organic reagents contain groups, which are reactive, i.e. capable of forming a covalent bond with a nucleophile. Typical electrophilic organic reagents include, for example, ethylene oxide, propylene oxide, butylene oxide, glycidol, 3-chioro-1,2-propanediol, methyl chloride, ethyl chloride, isatoic anhydride, succinicanhydride, octenylsuccinic anhydride, acetic anhydride, gamma-butyrolactone, beta-propiolactone, 1,3-propanesultone, acrylamide, glycidyltrimethyl ammonium chloride, glycidyldimethyl alkylammonium chloride such as lauryl, sodium chlorosulfonate, dimethyl sulfate, sodium chioroethanesulfonate, monochloroacetic acid, alkyl phenyl glycidyl ethers, glycidyl trimethoxysilanes, 1,2-epoxy dodecane and combinations thereof. One preferred class of electrophilic organic reagent includes those electrophilic organic reagents which contain an epoxide group, at least one acid group, preferably a diacid group and have from 3 to 18, preferably from 3 to 6 carbon atoms per molecule. Other preferred electrophilic organic reagents include cis-electrophilic organic reagents and trans-electrophilic organic reagent, with cis-electrophilic organic reagents being especially preferred. The electrophilic organic reagent may react with either the free amine or the underivatized hydroxyl groups of the chitosan. It is known that the amine functionality of the chitosan is generally regarded as a stronger nucleophilic site than the hydroxyl groups. Consequently weaker electrophilies will tend to react more readily with the amine groups than with the hydroxyl groups of the chitosan.

In addition further modified chitosan can be prepared which contain other substituent groups, such as hydroxyalkyl ether group (e.g., hydroxyethyl or hydroxypropyl ether groups), carboxyalkyl ether groups (e.g., carboxymethyl group), amide groups (e.g., succinyl groups), ester groups (e.g., acetate groups) or amino groups (e.g., 3-(trimethylammonium chloride) hydroxpropyl or 3-(dimethyloctadecylammonium chloride) hydroxpropyl ether groups) in addition to the electrophilic organic reagent groups. These other substituent groups maybe introduced prior to or subsequent to the reaction with the electrophilic organic reagent, or introduced simultaneously by reaction of the chitosan salt with the electrophilic organic reagent and the other derivatizing reagent.

Typically such covalently bonded chitosan derivative can be obtained by a process which includes the step of (a) dispersing a salt of chitosan (e.g., any one of those described herein before) in an effective amount of an aqueous caustic medium to form a neutralized chitosan containing free amine groups, (b) introducing an electrophilic organic reagent in the slurry and (c) maintaining the slurry at a temperature and time effective to promote the substitution of the electrophilic organic reagent onto the chitosan to form a covalently bonded chitosan derivative and the dissolution of the covalently bonded chitosan into the aqueous medium. The chitosan derivatives can be prepared in either salt form, i.e., ionically bonded, or in the covalently bonded from. Processes for the preparation of such chitosan derivatives are described in depth in US Pat No 5,621,088 issued on April 15. 1997 to Gruber and US Pat No 5,597,811 issued on January 28, 1997 to Gruber.

Suitable chitosans are commercially available from numerous vendors. Exemplary of a commercially available chitosan materials are those available from for example the Vanson Company.

One preferred chitosan salt for use herein is chitosan pyrrolidone carboxylate (also called chitosonium pyrrolidonecarboxylate), which has a degree of deacetylation of more than 85 %, a water solubility of 1 % (1 gram is soluble in 1 00 grams of distilled water at 25°C and one atmosphere), a pH of 4.5 and a viscosity between 100-300 cps. This chitosan pyrrolidone carboxylate is commercially available under the trade name Kytamero PC® from Amerchol Corporation. Another example of a suitable chitosan salt is chitosan pyrrolidone carboxylate available as Kytamer® PC from Amerchol Corporation of Edison, NJ. Other suitable FMAs are described in WO 01/80911.

Typically, the absorbent articles, such as disposable diapers, comprise a FMA or a mixture thereof at a level of from about 0.5 $g/m^2$ to about 500 $g/m^2$, preferably from about 1 $g/m^2$ to about 200 $g/m^2$, more preferably from about 3 $g/m^2$ to about 100 $g/m^2$ and most preferably from about 4 $g/m^2$ to about 50 $g/m^2$.

Table II shows the effects of concentration of various FMAs on the fecal analogs and/or feces. The mixing of the FMA and the fecal analog or feces is described below in the Sample Preparation Method.

TABLE II

| Fecal Analog | Chitin-based FMA | Concentration (wt. %) | Treated Feces/Fecal Analog Hardness (g force) |
|---|---|---|---|
| A | Chitosan[1] | 4.8 | 83 |
| A | Chitosan hydrochloride | 1.0 | 238 |
| A | Chitosan hydrochloride | 5.2 | 1430 |

1. A comecially available Chitosan, Hydagen HCMF available from Cognis Care Chemicals of Hoboken, NJ

While in certain embodiments it is desirable to treat the entire mass of feces within the article (i.e., "bulk" treatment), in some preferred embodiments only a portion of the feces is treated with the FMA. In these embodiments the FMA may penetrate only a relatively small distance into the feces, thereby forming a modified external layer that is relatively stiff and non-sticky. This may be preferable from an FMA utilization standpoint or to eliminate the need for mixing of the FMA into the fecal mass. The modified external layer is a region or layer of feces at or near the surface of the feces mass with different physical properties than the remainder of the feces mass. Preferably, the modified layer is harder (i.e., has a higher yield stress), less sticky, and/or has a higher resistance to diffusion of volatile molecules contained in the feces than do the remaining feces, resulting in decreased spreading/mobility of the feces' mass and/or decreased adhesion of the feces' mass to the wearer's skin and/or reduced fecal odor. Preferably, the modified external layer region is between about 1 and about 1000 microns in thickness and may cover all or any portion of the fecal mass. For example, it may be suitable to treat only the feces at the skin/feces interface (e.g., to reduce adhesion and/or promote cleaning or reduce spreading across the wearer's skin or to promote absorption or to reduce spreading within the article). Thus, to treat a 1 millimeter thick layer of a fecal mass over a 30 square cm area of the skin or article topsheet at a 10 weight percent level, 0.30 grams of the FMA must be available to the feces in the region of contact with the feces (assuming the specific gravity of the feces is 1.0).

In various embodiments, the FMA may be a liquid, solid (e.g., powder, fiber, film, web), or a semi-solid, or combinations thereof. The FMA may be presented in a water/oil or oil/water emulsion, a suspension, or mixture. The FMA may be disposed in the absorbent article as an individual discrete element (e.g., as a fibrous batt or layer within or attached to the absorbent article) or may be held in or on a carrier vehicle, such as a lotion or skin care composition, a web, or may be releasably encapsulated in a packet, cell, or envelope structure.

In embodiments wherein the FMA is delivered via a skin care composition, it may be soluble in the skin care composition or may be held in suspension or as a simple mixture.

The FMA may be delivered to the feces directly via transfer of the FMA to the feces or it may initially transfer to the wearer's skin or other element of the absorbent article prior to transfer to the feces. The carrier vehicle may be integral with the absorbent article or may constitute, or be component of, a separate absorbent article to be applied to the wearer (preferably at least over the perianal region) prior to, or in place of, a diaper, training pant, underwear, or other absorbent article.

When an optional carrier vehicle is present the FMA may be joined to the optional carrier vehicle using any means known in the art, such as adhesives (particularly water soluble adhesives), hydrogen bonding, releasable encapsulation, spraying, coating, and the like. Hydrogen bonding of the FMA to a absorbent article or component thereof may be effected by slightly wetting either the FMA or at least a portion of the substrate with water. Upon drying, the FMA is releasably affixed to the absorbent article (i.e., subsequent contact with liquid water will break the bond). This effect is enhanced for those FMAs which "gel" and become sticky when wet. Wetting may be accomplished by subjecting either the FMA, absorbent article, or both to a high humidity environment (e.g., 80% RH or greater) prior to or at the time of contact. Alternatively, water may be sprayed, misted, or atomized over at least a portion of either the FMA or absorbent article prior to or at the time of their contact. In such cases, the structure is preferably dried prior to incorporation into an absorbent article.

The FMA may contact the feces at or near the surface of the absorbent article (e.g., at the topsheet/feces interface), within the absorbent article (as in a waste management element), or at the body-side surface of the fecal mass (i.e., having first been transferred to the skin or other surface above the plane of the absorbent article). Typically, the FMA will contact the feces in the region of the absorbent article associated with the wearer's anus (e.g., crotch region in a diaper context). The feces may alternatively contact the FMA as it passes through an orifice, flange, valve, or the like, at or near the anus of the wearer. In such cases, the FMA may be expressed or drawn from the orifice or valve (e.g., from reservoirs) by the pressure of the passage of the feces as it extrudes from the body. The orifice may comprise a slit, slot, or perforation in a sheet, envelope, packet or other structure containing the FMA or composition comprising a FMA disposed in proximity to the exit point of the feces from the body. The orifice may be initially sealed by soluble film that is dissolved by contact with the feces, releasing the FMA or composition containing the FMA. Alternatively, the orifice may be opened as the structure is deformed by passage or pressure of the feces. The feces pressure, in addition to body pressure and movement may aid in the expression of FMA through the orifice to the feces.

In other alternative embodiments, the FMA may be associated with a gasket such as a leg cuff, waist barrier, waistband, waste pocket or with a feces spacing element. In embodiments wherein the FMA is associated with a gasketing element such as a leg cuff, waist barrier, or waist pocket, it is preferred that the FMA be associated with the portion of the gasket disposed closest to the exit point of the waste from the wearer (e.g., the anus).

The FMA may be delivered passively (e.g., the feces flows and contacts it during normal wearing conditions), actively (e.g., an element in the absorbent article responds to a signal and delivers/releases the FMA to the feces), or via a secondary carrier (e.g., a powder or other skin care composition initially transferred to the wearer's skin). Delivery of the FMA to the feces may occur as a result of feces extrusion pressure, weight, temperature, enzyme activity, water content, and/or pH; urine presence (e.g., urine triggering release of the FMA in response to or in anticipation of a defecation); body motions, pressure, or heat; or any other trigger or event during the wear cycle of the absorbent article.

The FMA may be initially stored within or on the absorbent article or any portion thereof and subsequently released by any of the triggering events described herein. In certain preferred embodiments, the FMA is releasably encapsulated under a film, in cells, packets, envelopes and the like so as to prevent migration and/or loss of the FMA prior to the absorbent article being insulted by feces and/or to aid in positioning the FMA for contact with the feces during use.

Optional Waste Management Element

In one embodiment of the present invention the absorbent article may include a waste management element. The waste management element is designed to help manage the acceptance, storage and/or immobilization of the viscous fluid bodily waste. The waste management element can be located anywhere in the absorbent article, including the crotch region or either waist region, or may be associated with or be included in any structure or element such as the core, a leg cuff, etc. In preferred embodiments, the waste management element is located in the region of the absorbent article that is near the wearer's perianal region when worn. This helps ensure that any waste discharged is deposited on or near the waste management element. The FMA of the present invention may be associated with any portion of the storage element, so long as it is in position to come into contact with feces.

The waste management element may be any material or structure capable of accepting, storing or immobilizing bodily exudates. Thus, the waste management element may include a single material or a number of materials operatively associated with each other. Further, the waste management element may be integral with another element of the absorbent article or may be one or more separate elements joined directly or indirectly with one or more elements of the absorbent article. Further, the waste management element may include a structure that is separate from the core or may include or be part of at least a portion of the core.

Suitable materials for use as the waste management element may include large cell open foams, macro-porous compression resistant nonwoven highlofts, large size particulate forms of open and closed cell foams (macro and/or microporous), highloft nonwovens, polyolefin, polystyrene, polyurethane foams or particles, structures comprising a multiplicity of vertically oriented looped strands of fibers, absorbent core structures described above having punched holes or depressions, and the like. (As used herein, the term "microporous" refers to materials which are capable of transporting fluids by capillary action. The term "macroporous" refers to materials having pores too large to effect capillary transport of fluid, generally having pores greater than about 0.5 mm in diameter and, more specifically, having pores greater than about 1.0 mm in diameter.) One embodiment of a waste management element includes a mechanical fastening loop landing element, having an uncompressed thickness of about 1.5 millimeters available as XPL-7124 from the 3M Corporation of Minneapolis, Minnesota. Another embodiment includes a resin-bonded nonwoven highloft comprising 6 denier, crimped fibers having a basis weight of 110 grams per square meter and an uncompressed thickness of 7.9 millimeters which is available from the Glit Company of Wrens, Georgia. Other suitable absorbent and nonabsorbent waste management element are described in U.S. Patent No. 5,941,864 entitled "Disposable Absorbent Article Having Improved Fecal Storage" issued to Roe on August 24, 1999. Further, the waste management element, or any portion thereof, may include or be coated with the FMA, a lotion or other known substances to add, enhance or change the performance or other characteristics of the waste management element.

Additionally the waste management element, when present, may comprise pockets for receiving and containing waste, spacers which provide voids for waste, barriers for limiting the movement of waste in the absorbent article, compartments or voids which accept and contain waste materials deposited in the absorbent article, and the like, or any combinations thereof. Examples of pockets and spacers for use in absorbent article as waste management element are described in U.S. Patent 5,514,121 to Roe et al.; U.S. Patent 5,171,236 to Dreier et al.; U.S. Patent 5,397,318 to Dreier; U.S. Patent 5,540,671 to Dreier; PCT Application WO 93/25172 published December 3, 1993 entitled "Spacers For Use In Hygienic Absorbent Articles And Disposable Absorbent Articles Having Such Spacer"; U.S. Patent 5,306,266 to Freeland; and U.S. Patent 5,997,520 to Ahr et al.. Examples of compartments or voids are disclosed in U.S. Patent 4,968,312 to Khan; U.S. Patent 4,990,147 to Freeland; U.S. Patent 5,062,840, to Holt et al; and U.S. Patent 5,269,755 to Freeland et al. Examples of suitable transverse barriers are described in U.S. Pat. No. 5,554,142 to Dreier et al.; PCT Patent WO 94/14395 entitled "Absorbent Article Having An Upstanding Transverse Partition" published July 7, 1994 in the name of Freeland, et al.; and U.S. Patent No. 5,653,703 to Roe, et al. Examples of other structures especially suitable for management of low viscosity feces are disclosed in U.S. Patents 5,941,864 to Roe et al.; U.S. Patent No. 5,977,430 to Roe et al. and 6,013,063 issued to Roe et al.

The FMAs may also be held on or within macro-particulate elements. These macro-particulate elements may be contained in a waste management element attached to a topsheet, cuff, or other feature of the absorbent article (releasably or not), or loose in a separate absorbent article attached independently to the body. Further, any of the structures that hold, carry, deliver, or mix the FMA may comprise protrusions or other three-dimensional geometries designed to increase contact area of the FMA and the feces and/or to promote mixing.

Those embodiments of the absorbent article of the present invention which include a waste management element, may optionally have waste management elements having an Acceptance Under Pressure of greater than about 0.5 g of viscous fluid bodily waste per square inch of the waste management element per mJ (milliJoule) energy input. More preferably, the waste management element should have an Acceptance Under Pressure of greater than about 0.6 grams per square inch per milliJoule of energy of viscous fluid bodily waste. Even more preferably, the waste management element should have an Acceptance Under Pressure of greater than about 0.8 grams per square inch per milliJoule of energy, and most preferably greater than about 1.0 grams per square inch per milliJoule of energy of viscous fluid bodily waste. Generally, Acceptance Under Pressure values between at least about 0.6 grams per square inch per milliJoule of energy and about 10.0 grams per square inch per milliJoule of energy, and between about 0.8 grams per square inch per milliJoule of energy and about 10.0 grams per square inch per milliJoule of energy have been found to be acceptable.

Alternatively, the waste acceptance element may optionally have a Receptivity Under Pressure of at least about 1.5 grams of viscous fluid bodily waste per square inch of the waste management element per milliWatt (mW) of power, more preferably greater than about 3.0 grams per square inch per milliWatt of power, even more preferably greater than about 5.0 grams per square inch per milliWatt of power, most preferably greater than about 10.0 grams per square inch per milliWatt of power. Generally, the Receptivity Under Pressure is between about 1.5 and about 50.0 grams per square inch per milliWatt of power and may be between about 5.0 and about 50.0 grams per square inch per milliWatt of power.

These preferred Acceptance and Receptivity Under Pressure parameters relate to integrated absorbent articles which are preferably evaluated as they are intended for use. That is, if the absorbent article intended for use comprises more than one component or layer, all of the components or layers of the absorbent article should be configured as they would be during normal use when the measurement of their performance is made. A more detailed description of the method for determining Acceptance Under Pressure performance is included in the Test Methods section, below.

TEST METHODS

Viscosity

The viscosity analog or feces may be determined by a controlled stress rheometer. A suitable rheometer is available from T. A. Instruments, Inc. of New Castle, DE, as model number $SC^2100$. The rheometer utilizes a stainless steel parallel plate fixture. The rheometer has a rigid horizontal first plate onto which the sample is placed and a second plate mounted over the first plate such that the axis of said second plate is perpendicular to the first plate. The second plate is 2 or 4 centimeters in diameter. A two centimeter (2 cm) parallel plate is used for firm, pasty, or highly mucousy samples, while the four centimeter (4 cm) parallel plate is used for very runny or "water-like" feces or analog samples. The first and second plates are spaced apart up to 2000 microns during the measurement process. The second plate is connected to a drive shaft for axial rotation. The drive motor and strain sensor are also mounted on the drive shaft. All measurements are to be conducted at standard temperature, pressure and humidity, that is 70 °F, 1 atmosphere, and 50% humidity.

A suitable sample (typically 2 to 3 grams) of an analog or feces to be tested is centered on the first plate and generally centered beneath the axis of the second plate. Prior to the test, any large pieces of undigested food material (e.g., seeds) are removed. The first plate is raised into position. Excess amounts of the sample which are displaced beyond the diameter of the second plate are removed using a spatula. Water is then misted around the edges of the sample to prevent edge effects due to moisture loss during the measurement process. A programmed application of a shear stress, from 50 to 50,000 dynes/cm$^2$ for pasty and firm samples, is applied to the sample by the rheometer. For runny and watery samples, a shear stress range of 5 to 5000 dynes/cm$^2$ is used. The data is fitted to a power law function where the apparent viscosity = k $j^{(n-1)}$, k = consistency (units of cP x sec$^{(n-1)}$, j = shear rate (Units of 1/sec), and n = shear index (dimensionless). Therefore, when j = one 1/sec, the viscosity = k. (The plates are maintained at 35 °C throughout the test.)

Hardness Method

Hardness is measured using a Stevens-Farnell QTS-25 Texture Analyzer, model 7113-5kg, and associated software on an Intel-based machine having a 486 processor or higher. A ½ inch stainless steel spherical probe and an analog receptacle are provided. A suitable probe is the TA18 probe available from Leonard Farnell Co. of Hatfield, England. The analog receptacle can be made by cutting a 7 milliliter linear low density polyethylene scintillation vial (having an inside diameter of 0.55 inches +/- 0.005 inches) to about 16 millimeter length. Suitable vials are available from Kimble Glass Company of Vineland, New Jersey as #58503-7 vials. The analog receptacle is filled to the top edge (level) with the analog (Analog A or Analog B, as described herein) or feces to be tested. If a modification agent is to be evaluated, the sample is prepared via the Sample Preparation Method described below. The vial is centered under the ½ inch spherical stainless steel probe. The probe is lowered such that it just contacts the surface of the analog in the vial. The probe is moved downward 7 millimeters at about 100 millimeters per minute and then stopped. The Hardness is the maximum recorded resistive force in grams encountered by the probe on its 7 millimeter stroke. (The temperature of the room and the analog or feces should be between about 65 to 75 °F during the course of the measurement.) For reference, Hardness has been found to relate strongly to the complex modulus of the material, which is a combination of the viscous and elastic moduli of the material.

Method for Making Analog A

1.5 grams of ULTRA DAWN DISHWASHING LIQUID (available from the Procter & Gamble Co, Cincinnati, OH) is added to an empty metal mixing bowl. 10 grams each of Feclone FPS-2 and Feclone FPS-4 are added into the bowl containing the Dawn. (Both Feclone materials are available from Siliclone Studios, Valley Forge, PA.) Then 200 milliliters of distilled water, heated to 200°F, is added to the mixing bowl. The resultant mixture is then carefully stirred by hand, to avoid introducing air bubbles to the mixture, using a rubber or plastic spatula until homogenous, (usually about 3-5 minutes). When prepared properly, the Analog A will have a Hardness between about 7 and about 10 grams force as measured by the above Hardness Method.

Method for Making Analog B

Viscous fluid bodily waste analog, Analog B, is a fecal material analog made by mixing 10 grams of Carbopol 941 available from the B.F. Goodrich Corporation of Brecksville, OH, or an equivalent acrylic polymer in 900 milliliters of distilled water. The Carpobol 941 and distilled water are weighed and measured separately. A 3-bladed marine-type propeller having a 2 inch diameter paddle (available from VWR Scientific Products Corp. of Cincinnati, Ohio, Catalog # BR4553-64, affixed to a 3/8" stirring shaft BR4553-52) is used to stir the distilled water. The propeller speed should be constant at 450 rpm during mixing. The mixer should form a vortex without splashing. The Carbopol is slowly sieved into the water so that it is drawn into the vortex and mixed without forming white clumps, or "fish eyes". The mixture is stirred until all of the Carbopol has been added, and then for a period of 2 minutes thereafter. The sides of the bowl containing the mixture should be scraped and the bowl should be rotated as needed to achieve a homogeneous mixture. (The mixture will likely be slightly cloudy with air bubbles). One hundred grams of a 1.0 N volumetric NaOH solution, available from J. T. Baker Co., Phillipsburg, NJ, is then slowly measured into the mixture and the mixture is stirred until homogeneous. The mixture should become thick and clear. The mixture should be stirred for 2 minutes after the addition of the alkali solution. The neutralized mixture should be allowed to equilibrate for at least 12 hours and should be used for the Acceptance Under Pressure test within 96 hours thereafter. Before the Analog B mixture is used, it should be stirred in the container at low speed (about 50 rpm) for about 1 minute to ensure the mixture is homogeneous,

Analog B should, when prepared correctly, have a "hardness" value between 55 and 65 grams. Hardness is measured using a Stevens-Farnell QTS-25 Texture Analyzer, model 7113-5kg, and associated software on an Intel-based machine having a 486 processor or higher. A ½ inch stainless steel spherical probe and an analog receptacle are provided. A suitable probe is the TA18 probe available from Leonard Farnell Co. of Hatfield, England. The analog receptacle can be made by cutting a 7 milliliter linear low density polyethylene scintillation vial (having an inside diameter of 0.55 inches +/- 0.005 inches) to a 15 millimeter length. Suitable vials are available from Kimble Glass Company of Vineland, New Jersey as #58503-7 vials. The analog receptacle is filled to within 2 millimeters of the top edge with the analog to be tested. The vial is centered under the ½ inch spherical stainless steel probe. The probe is lowered to a distance of about 1 millimeter from the surface of the analog in the vial. The probe is moved downward 7 millimeters at 100 millimeters per minute and then stopped. The Hardness is the maximum recorded resistive force encountered by the probe on its 7 millimeter stroke. (The temperature of the room and the analog should be between about 65 to 75 °F during the course of the measurement.)

The reference Hardness value of a synthetic fecal analog material, Fecal Analog A is 9.7 g of force. Analog A represents the water content, Hardness, and adhesion properties of typical "runny" feces.

The effect of mixing several comparative examples with Analog A are illustrated in Table I below. All comparative materials were mixed with the Analog A. It is clear the desired changes in Hardness were not achieved by the comparative materials.

TABLE I

| Fecal Analog | Comparative Additive | Concentration (wt.%) | Treated Fecal Analog Hardness (g force) |
|---|---|---|---|
| A | Corn Starch[1] | 1.0 | 12.6 |
| | | 5.0 | 8.6 |
| A | Pure Corn Starch Baby Powder[2] | 1.0 | 14.4 |
| | | 5.0 | 7.1 |
| A | Baby Powder[2] | 1.15 | 10.2 |

1. Dietary Fiber Control, Sigma Chemical Co., St. Louis, MO, S-2388.
2. Johnson & Johnson, Co., Skillman, NJ

Acceptance and Receptivity Under Pressure

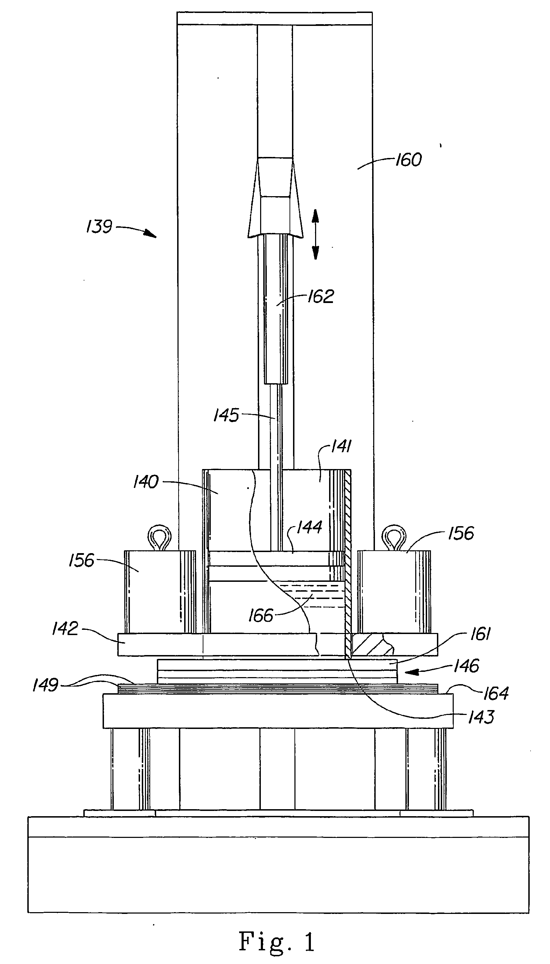

Acceptance Under Pressure is measured by the following test which uses the apparatus 139 illustrated in Figure 1. A hollow Plexiglas cylinder 140 is provided mounted on a stainless steel plate 142 about 9.5 mm thick. Plate 142 is a square, about 10.16 cm x 10.16 cm (about 4 in. x 4 in.). The cylinder 140 and plate 142 combination has a height of 7.6 centimeters (about 3.0 inches), an inside diameter of 5.08 centimeters (about 2.00 inches) and an outside diameter of 6.3 centimeters (about 2.48 inches). The bottom of cylinder 140 extends below plate 142 a distance of about 3.5 millimeters. Lip 143 prevents test feces or analog 166 from leaking outside the designated test area. Two 625 gram weights 156 are also provided, each having a diameter of 5.08 cm (about 2.0 inches).

A cylindrically shaped 24.6 gram Plexiglas weight 144 is provided. Weight 144 has a diameter of 5.08 centimeters (about 2.0 inches), so that weight 144 fits with close tolerance within cylinder 140 but can freely slide throughout hole 141 in cylinder 140. This arrangement provides a pressure of about 119 Pascals (Pa) (about 0.017 pounds per square inch) and a test area of about 20.27 square cm (about 3.142 square inches). If desired, weight 144 may have a handle 145 to allow it to be easily inserted into and removed from the cylinder 140. In such cases, the combined mass of handle 145 and cylindrical weight 144 should equal 24.6 grams.

A sample 146 of the structure to be tested for Acceptance Under Pressure properties is provided. The sample 146 may be cut from an existing diaper or may be constructed from material which has not been formed into a diaper. Sample 146 includes the entire structure intended for use in an article or the entire structure of the article to be evaluated, including top layer 161. Sample 146 should be cut into a square measuring 10.16 centimeters by 10.16 centimeters (about 4 inches by 4 inches).

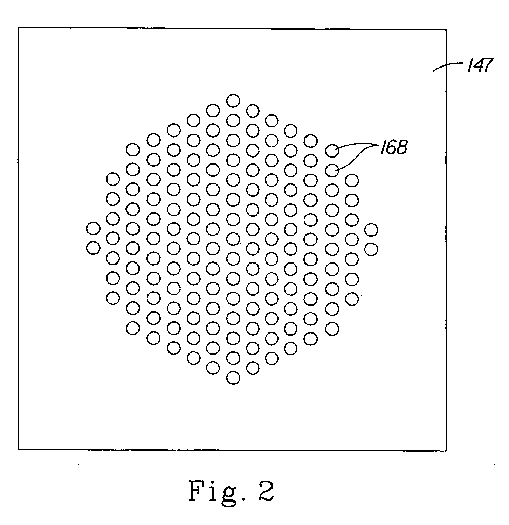

In order to measure the Acceptance Under Pressure performance of discrete waste management elements, as described herein, the Acceptance Under Pressure test is performed using the standard storage element 147, as shown in Figure 2, in place of any underlying structure or layers. The standard storage element 147 includes a 4 inch square 1.6 millimeter thick aluminum plate having a pattern of 153 regularly spaced 4.3 millimeter diameter holes 168, as shown in Figure 2. The holes are arranged such that there are approximately 26 holes per square inch)

Five sheets of a high basis weight blotter 149 measuring 4 inches x 4 inches are provided. The blotter material 149 is preferably filtration grade paper, available from Ahlstrom Filtration, Inc. of Mt. Holly Springs, Pennsylvania as #632-025, having a basis weight of about 90 grams per meter$^2$.

The top layer 161 of sample 146 is removed and the remaining components, or layers, of sample 146 (if there are multiple components or layers) and the five sheets of blotter material 149 are weighed to the nearest 0.01 grams. Thus, if sample 146 is being taken from a diaper, the layers of the diaper such as topsheets, secondary topsheets, acquisition layers, absorbent cores etc., should be separated prior to weighing. (In some cases, a single layer may comprise two or more permanently bonded components.) In so doing, care must be taken not to destroy the sample 146 or cause unintended gross deformation of any parts of the sample 146. The layers of the sample 146 may be frozen to aid their separation from adjacent layers of the sample 146. Freezing may be accomplished using PH100-15 circuit refrigerant made by Philips ECG, Inc. of Waltham, Massachusetts.

The sample 146 is then reassembled as originally configured on top of 5 stacked sheets of blotter material 149 with the side of the sample 146 intended to face the wearer oriented facing up and away from the blotter material 149.

The combined assembly of the sample 146 and the blotter material 149 is centered on the work surface 164 of a Stevens-Farnell QTS-25 Model 7113-5kg Texture Analyzer 160 (available from Leonard Farnell Co. of Hatfield, England), under the probe 162. A suitable probe 162 is a 100 cm flat-ended cylindrical aluminum extension rod "QTSM3100" available from the Leonard Farnell Co. of Hatfield England. The cylinder 140 is centered on the sample 146. The two 625 gram weights 156 are placed on opposite corners (diagonally) of the plate 142 to stabilize it. A syringe having an opening of about 4 to 6 millimeters is used to dispense approximately 10 cubic centimeters of viscous fluid bodily waste analog 166 (Analog B as described herein) through the hole 141 in the cylinder 140 onto the top of the sample 146.

Once the proper amount of viscous fluid bodily waste analog 166, Analog B, has been measured into the cylinder 140, the 24.6 gram weight 144 is inserted slowly and gently into the hole 140 in the cylinder 140 until it rests on the surface of the analog. The Texture Analyzer 160 is activated so the probe 162 depresses the cylindrical weight 144 at a rate of 10 millimeters per minute until a resisting force of about 144.6 grams is reached. The Texture Analyzer 160 is set to stop the downward stroke once the resistance force of 144.6 grams is reached. The recorder is set to trigger at a resistive force of 5 grams. (The maximum resisting force of 144.6 grams corresponds to an applied pressure of 700 Pascals or 0.1 pounds per square inch). Once a resistive force of 144.6 grams is reached, the probe 162 is retracted to its starting position.

The weight 144 is removed from the cylinder 140, and then the cylinder 140 is removed from the surface of the sample 146, taking care not to drip any Analog B remaining in the cylinder 140 onto the sample. The top layer 161 of the sample 146 is then removed from the underlying layer(s) of the sample 146 by dragging the top layer 161 parallel to the surface of the underlying layers, if possible. For certain structures where the top layer 161 is difficult to remove by dragging parallel to the underlying layers, the top layer 161 may be peeled or lifted away from the underlying layers of sample 146. If the sample 146 comprises only a single layer, the standard acceptance element 151, described below, is utilized as the top layer 161 of the sample 146. The underlying layers of the sample 146 and the blotter material 149 are then weighed. The amount of test Analog B accepted by the sample 146 equals the increase in combined weight of the underlying layer(s) of the sample 146 and the blotter material 149 caused by the test Analog B penetrating through the top surface layer of the sample 146 per unit work performed (in milliJoules) on a unit area basis. The area under the force vs. distance curve, used in calculating the unit work, is calculated by integrating the force resisting the probe on its downward stroke over the total distance traveled until the maximum force of 144.6 grams is registered. The unit work is calculated using the following equation:

$$\text{Unit Work (mJ)} = \text{Area under the force vs. distance curve (g/mm)} \ (9.81 \ \text{m/s}^2)/(1000 \ \text{mm/m})$$

The Acceptance Under Pressure is calculated using the following equation:

$$\text{Acceptance Under Pressure} = \frac{\text{amount of analog in structure (g)} \div \text{work required (mJ)}}{\text{Test area (in}^2)}$$

Receptivity Under Pressure is measure in the same manner as Acceptance Under Pressure, as described above, except that the time required to reach the resistive force of 144.6 grams on the downward stroke of the probe 162 is measured and recorded. Receptivity Under Pressure is calculated using the following equation:

$$\text{Receptivity Under Pressure (g/in}^2\text{/mW)} = \frac{\text{Acceptance Under Pressure (g/in}^2\text{/mJ)}}{\text{Time required to Reach 144.6g Resistive force (sec)}}$$

Method for Making Chitosan Hydrochloride

An aqueous hydrochloric acid (HCl) solution is made by mixing 5.39g of 1.0 $\underline{M}$ HCL in 1057 ml of deionized water. Subsequently, 10.72g of Chitosan (Hydagen HCMF) is added to the above aqueous HCL solution, and stirred vigorously at room temperature (22° C) until all the chitosan is dissolved, resulting in a clear solution. The resultant mixture is then poured evenly into an aluminum sheet pan, (e.g., Model# 9002, Lincoln Foodservice Products, 1111 North Hadley Road, Fort Wayne, IN. 48604) having a length of 25.75 inches, a width of 17.75 inches wide, and a depth of 1 inch. The pan is maintained in a level orientation (i.e., the plane defined by the bottom surface of the sheet is held normal to the gravitational direction) while the solution is in a liquid state. The pan and contents are then placed in a fume hood to dry at room temperature (i.e., in the range of about 22° - 25° C) for 36 hours. When dry, the chitosan hydrochloride remains in the pan as a film that is easily removed from the pan. The film is removed from the pan and is manually torn into smaller pieces having dimensions between about 1 cm X 1 cm and 10 cm X 10 cm that are placed in a second aluminum pan 8 inches long, 8 inches wide, and 2 inches deep. The pan and contents are heated in a convection oven for 24 hours at 110° F to produce dry, brittle sheets. The resultant brittle sheets are then manually broken into smaller pieces no larger than 5 cm in their largest dimension. These smaller pieces of brittle film are broken into smaller flakes using a coffee grinder (e.g., Braun Automatic Coffee Grinder Model# KSM4B from the Braun US Division, The Gillette Company of Boston, MA), the largest flakes having an area of about 9 mm$^2$ and a thickness of about 0.5 mm. The average flake is about 1 to 2 mm square and has a thickness of about 0. 1 to about 0.4 mm.

Sample Preparation Method

A 250 mL Precleaned VWRbrand TraceClean jar (VWR # 15900-196) is placed on a balance and tared. The desired amount of FMA is measured into the cup and its exact weight is recorded. After the FMA's weight is recorded the balance is tared again. The desired amount of feces or analog is measured into the cup containing the FMA. The exact amount of feces or analog is recorded and the FMA and feces or analog is stirred vigorously using the spatula end of a Standard Ayre Cervi-Scraper (VWR # 15620-009) until homogeneous (total stirring time is generally about 2 minutes). For the purposes of this method, the beginning of the stirring process is defined as t = 0 minutes. After the sample is mixed it is allowed to sit for the remainder of the desired reaction time. For the data presented herein, this reaction time is set at t = three minutes elapsed from the beginning of the stirring process. It is then loaded into the 16mm receptacle described above in the Hardness Method using the spatula end of a Standard Ayre Crevi-Scraper, and the Hardness test is performed (starting at t = 3 min. from the beginning of the stirring process, as described above).

**Claims**

1. An absorbent article adapted to receive feces having a first waist region, a second waist region opposed to said first waist region, a crotch region disposed between said first waist region and said second waist region, said absorbent article comprising:

   (a) a liquid pervious topsheet;
   (b) a liquid impervious backsheet joined to at least a portion of said topsheet;
   (c) an absorbent core disposed between at least a portion of said topsheet and said backsheet, and
   (d) an effective concentration of a chitin-based feces modifying agent disposed in said article such that said chitin-based feces modifying agent is available to contact at least a portion of said feces deposited in said article, whereby changing the viscosity or Hardness of at least some of the feces which may be deposited in the article,

wherein said chitin-based feces modifying agent is associated with a gasket, such as a leg cuff, waist barrier, waistband, waste pocket or with a feces spacing element.

2. An absorbent article adapted to receive feces having a first waist region, a second waist region opposed to said first waist region, a crotch region disposed between said first waist region and said second waist region, said absorbent article comprising:

(a) a liquid pervious topsheet;
(b) a liquid impervious backsheet joined to at least a portion of said topsheet;
(c) an absorbent core disposed between at least a portion of said topsheet and said backsheet;
(d) a waste management element having an Acceptance Under Pressure value of greater than 0.50 grams per square inch per milliJoule of energy; and
(e) an effective concentration of a chitin-based feces modifying agent

wherein said feces deposited in said article has a hardness and said hardness is increased by greater than or equal to 100% by said chitin-based feces modifying agent at a concentration of no more than 5% by weight of the article.

3. The article of any one of Claims 1 or 2 wherein said chitin-based feces modifying agent is selected from the group consisting of chitosan, chitosan salts, chitosan derivatives, modified chitosan and mixtures thereof.

4. The article of Claim 1 wherein said feces deposited in said article has a hardness and said Hardness is increased by greater than or equal to 100% by said chitin-based feces modifying agent at a concentration of no more than 5.0 % by weight of the article.

5. The article of any one of Claims 1 to 3 wherein said feces Hardness is increased by greater than or equal to 100% by said chitin-based feces modifying agent at a concentration of no more than 1.0 % by weight of the article.

6. The article of any one of Claims 1 to 3 wherein said chitin-based feces modifying agent is present in concentration of greater than or equal to 0.001% by weight of the article.

7. The article of any one of Claims 1 to 6 wherein said chitin-based feces modifying agent is chitosan hydrochloride.

**Patentansprüche**

1. Absorptionsartikel, der zur Aufnahme von Fäkalien ausgelegt ist, mit einem ersten Taillenbereich, einem zweiten Taillenbereich gegenüber dem ersten Taillenbereich, einem Schrittbereich, der zwischen dem ersten Taillenbereich und dem zweiten Taillenbereich angeordnet ist, wobei der Absorptionsartikel Folgendes umfasst:

(a) eine flüssigkeitsdurchlässige Oberschicht;
(b) eine flüssigkeitsundurchlässige Unterschicht, die mit mindestens einem Teil der Oberschicht verbunden ist;
(c) einen Absorptionskern, der zwischen mindestens einem Teil der Oberschicht und der Unterschicht angeordnet ist; und
(d) eine wirksame Konzentration eines Fäkalien modifizierenden Mittels auf Chitinbasis, das so in dem Artikel angeordnet ist, dass das Fäkalien modifizierende Mittel auf Chitinbasis für den Kontakt mit mindestens einem Teil der Fäkalien, die in den Artikel ausgeschieden werden, verfügbar ist, wodurch die Viskosität oder Härte von mindestens einem Teil der Fäkalien, die in den Artikel ausgeschieden werden können, verändert wird, wobei das Fäkalien modifizierende Mittel auf Chitinbasis mit einer Dichtung verbunden ist, wie einem Beinbündchen, einer Taillensperrschicht, einem Taillenbund, einer Exkremententasche oder einem Fäkalienabstandselement.

2. Absorptionsartikel, der zur Aufnahme von Fäkalien ausgelegt ist, mit einem ersten Taillenbereich, einem zweiten Taillenbereich gegenüber dem ersten Taillenbereich, einem Schrittbereich, der zwischen dem ersten Taillenbereich und dem zweiten Taillenbereich angeordnet ist, wobei der Absorptionsartikel Folgendes umfasst:

(a) eine flüssigkeitsdurchlässige Oberschicht;

(b) eine flüssigkeitsundurchlässige Unterschicht, die mit mindestens einem Teil der Oberschicht verbunden ist;

(c) einen Absorptionskern, der zwischen mindestens einem Teil der Oberschicht und der Unterschicht angeordnet ist;

(d) ein Element zum Umgang mit Exkrementen, mit einem Wert der Aufnahme unter Druck von mehr als 0,50 Gramm pro 6,5 Quadratzentimeter (Quadratinch) pro Millijoule Energie; und

(e) eine wirksame Konzentration eines Fäkalien modifizierenden Mittels auf Chitinbasis, wobei die Fäkalien, die in den Artikel ausgeschieden werden, eine Härte aufweisen und die Härte durch das Fäkalien modifizierende Mittel auf Chitinbasis in einer Konzentration von nicht mehr als 5 Gew.-% des Artikels um mehr als oder gleich 100 % erhöht wird.

3. Artikel nach einem der Ansprüche 1 oder 2, wobei das Fäkalien modifizierende Mittel auf Chitinbasis ausgewählt ist aus der Gruppe, bestehend aus Chitosan, Chitosansalzen, Chitosanderivaten, modifiziertem Chitosan und Mischungen davon.

4. Artikel nach Anspruch 1, wobei die Fäkalien, die in den Artikel ausgeschieden werden, eine Härte aufweisen und die Härte durch das Fäkalien modifizierende Mittel auf Chitinbasis in einer Konzentration von nicht mehr als 5,0 Gew.-% des Artikels um mehr als oder gleich 100 % erhöht wird.

5. Artikel nach einem der Ansprüche 1 bis 3, wobei die Fäkalienhärte durch das Fäkalien modifizierende Mittel auf Chitinbasis in einer Konzentration von nicht mehr als 1,0 Gew.-% des Artikels um mehr als oder gleich 100 % erhöht wird.

6. Artikel nach einem der Ansprüche 1 bis 3, wobei das Fäkalien modifizierende Mittel auf Chitinbasis in einer Konzentration von mehr als oder gleich 0,001 Gew.-% des Artikels vorhanden ist.

7. Artikel nach einem der Ansprüche 1 bis 6, wobei das Fäkalien modifizierende Mittel auf Chitinbasis Chitosanhydrochlorid ist.

## Revendications

1. Article absorbant adapté pour recevoir des matières fécales ayant une première région de ceinture, une deuxième région de ceinture opposée à ladite première région de ceinture, une région d'entrejambe disposée entre ladite première région de ceinture et ladite deuxième région de ceinture, ledit article absorbant comprenant :

(a) une feuille de dessus perméable aux liquides ;

(b) une feuille de fond imperméable aux liquides attachée à au moins une partie de ladite feuille de dessus ;

(c) une âme absorbante disposée entre au moins une partie de ladite feuille de dessus et de ladite feuille de fond ; et

(d) une concentration efficace d'un agent de modification des matières fécales à base de chitine disposé dans ledit article de telle sorte que ledit agent de modification des matières fécales à base de chitine est disponible pour entrer en contact avec au moins une partie desdites matières fécales déposées dans ledit article, modifiant ainsi la viscosité ou la dureté d'au moins certaines des matières fécales qui sont déposées dans l'article, dans lequel ledit agent de modification des matières fécales à base de chitine est associé à un joint statique, par exemple un revers de jambe, une barrière de ceinture, une ceinture, une poche à déchets ou avec un élément d'espacement des matières fécales.

2. Article absorbant adapté pour recevoir des matières fécales ayant une première région de ceinture, une deuxième région de ceinture opposée à ladite première région de ceinture, une région d'entrejambe disposée entre ladite première région de ceinture et ladite deuxième région de ceinture, ledit article absorbant comprenant:

(a) une feuille de dessus perméable aux liquides ;

(b) une feuille de fond imperméable aux liquides attachée à au moins une partie de ladite feuille de dessus ;

(c) une âme absorbante disposée entre au moins une partie de ladite feuille de dessus et de ladite feuille de fond ;

(d) un élément de prise en charge des déchets ayant une valeur d'acceptation sous pression supérieure à 0,50 grammes par 6,5 centimètres carrés (pouce carré) par milliJoule d'énergie ; et

(e) une concentration efficace de matières fécales à base d'un agent de modification des matières fécales à base de chitine dans lesquelles lesdites matières fécales déposées dans ledit article ont une dureté et ladite dureté est augmentée d'une valeur supérieure ou égale à 100 % par ledit agent de modification des matières fécales à base de chitine à une concentration de pas plus de 5 % en poids de l'article.

3. Article selon l'une quelconque des revendications 1 ou 2, dans lequel ledit agent de modification des matières fécales à base de chitine est choisi dans le groupe constitué de chitosan, sels de chitosan, dérivés de chitosan, chitosan modifié et leurs mélanges.

4. Article selon la revendication 1, dans lequel lesdites matières fécales déposées dans ledit article ont une dureté et ladite dureté est augmentée d'une valeur supérieure ou égale à 100 % dudit agent de modification des matières fécales à base de chitine à une concentration de pas plus de 5,0 % en poids de l'article.

5. Article selon l'une quelconque des revendications 1 à 3, dans lequel ladite dureté des matières fécales est augmentée d'une valeur supérieure ou égale à 100 % dudit agent de modification des matières fécales à base de chitine à une concentration de pas plus de 1,0 % en poids de l'article.

6. Article selon l'une quelconque des revendications 1 à 3, dans lequel ledit agent de modification des matières fécales à base de chitine est présent en une concentration supérieure ou égale à 0,001 % en poids de l'article.

7. Article selon l'une quelconque des revendications 1 à 6, dans lequel ledit agent de modification des matières fécales à base de chitine est du chlorhydrate de chitosan.

Fig. 1

Fig. 2